# EUROPÄISCHE PATENTSCHRIFT

(11) **EP 1 846 067 B1**
(45) Veröffentlichungstag und Bekanntmachung des Hinweises auf die Patenterteilung: **14.10.2009**
(21) Anmeldenummer: 06791584.3
(22) Anmeldetag: 17.08.2006
(51) Int. Cl.: A61M 5/315, A61M 5/32, A61M 5/20

(54) **AUTOINJEKTIONSGERÄT**
AUTOMATIC INJECTOR DEVICE
DISPOSITIF D'AUTO-INJECTION

(30) Priorität: 17.08.2005 DE 102005038933
(43) Veröffentlichungstag der Anmeldung: 24.10.2007
(73) Patentinhaber: Peter Loos und Arnold Neuhold Gewerbliche Schutzrechte GbR, 82418 Murnau (DE)
(72) Erfinder: NEUHOLD, Arnold, 82386 Huglfing (DE)
(74) Vertreter: HOFFMANN EITLE
(86) Internationale Anmeldenummer: PCT/EP2006/008123
(87) Internationale Veröffentlichungsnummer: WO 2007/020090

(56) Entgegenhaltungen:
- EP-A2- 0 956 875

## Beschreibung

Die Erfindung bezieht sich auf ein Autoinjektionsgerät, insbesondere zum Injizieren von Produkten in den Körper eines Lebewesens, mit einem Gehäuse, einem darin befindlichen, das zu injizierende Produkt enthaltenden Behälter, der mit einer Injiziernadel in Verbindung steht und einen Injizierkolben enthält, mit einer Antriebseinrichtung mit wenigstens einem Antriebselement und wenigstens einem Abtriebselement, wobei das Antriebselement auf das Abtriebselement und das Abtriebselement auf den Injizierkolben und/oder den Behälter mit der daran befindlichen Injiziernadel einwirkt, mit einer Dämpfungseinrichtung zumindest zum Dämpfen des Einstechvorganges und/oder der Injizierbewegung des Injizierkolbens, wobei das Abtriebselement bis zum Erreichen einer vorderen Position des Behälters vom Injizierkolben vorschubmäßig entkoppelt und über ein Kopplungs- und Dämpfungselement in Antriebsrichtung mittelbar oder unmittelbar mit dem Behälter gekoppelt ist und das Kopplungs- und Dämpfungselement zur Entkopplung an einer Kopplungsstelle einen Widerstand überwinden muss, und bei Erreichen der vorderen Position mit dem Injizierkolben vorschubmäßig gekoppelt wird, und wobei das Kopplungs- und Dämpfungselement zur Dämpfung mit einem Dämpfungs-Gegenelement zusammen wirkt.

Ein derartiges Gerät ist beispielsweise aus der DE 198 21 933 C1, der EP 0 956 875 A2 die ein gattungsgemäßes Autoinjektionsgerät offenbart, und der DE 198 22 031 C2 bekannt. Dabei handelt es sich um sogenannte Injektionspens. Durch das Antriebselement erfolgt nach einer Auslösung zunächst mit der Bewegung des Behälters ein Einstechen der Injektionsnadel beispielsweise in die Haut eines menschlichen Körpers. Nach einem Anschlag am Gehäuse erfolgt die Überwindung des erwähnten Widerstandes mit der vorstehend kurz beschriebenen Entkopplung, so dass das Abtriebselement, angetrieben vom Antriebselement, den Injizierkolben im Behälter vorschiebt, so dass das im Behälter befindliche Produkt injiziert wird. Danach erfolgt eine Dämpfung des Injektionsvorganges als kontrollierte Dämpfung im Hinblick eines kontinuierlichen Injizierens. Im Zusammenhang mit der Entkopplung ist nicht nur der erwähnte Widerstand zu überwinden, sondern auch die hohe Reibung des Injizierkolbens innerhalb des Behälters, wozu ein Kraftaufwand bis zu 20 N notwendig sein kann, abhängig beispielsweise von der Zeit der Lagerung des Autoinjektionsgerätes und der Herstellungstoleranzen des Behälters, der üblicherweise aus Glas besteht. Somit erfolgt bei dem bekannten Gerät schon eine kontrollierte Dämpfung zur Erzeugung einer konstanten Vorschubgeschwindigkeit des Injizierkolbens und einer konstanten Produktabgabe. Allerdings ist nicht vermeidbar, dass bei der ruckartigen Entkopplung zu Beginn des Injiziervorganges sehr abrupt und schnell das Produkt in die Haut eines menschlichen Körpers injiziert wird, so dass Hämatome entstehen können.

Ein Autoinjektionsgerät in der hier in Rede stehenden Art ist auch aus der WO 2004/098687 A1 der Anmelderin bekannt, worauf die vorliegende Erfindung ausdrücklich Bezug nimmt.

Gemäß den erstgenannten Druckschriften zum Stand der Technik ist vorgesehen, die Dämpfung pneumatisch oder mechanisch durch Reibung vorzunehmen.

Es ist das zu lösende Problem (Aufgabe) der vorliegenden Erfindung, ein Autoinjektionsgerät der Eingangs genannten Art zu schaffen, mit dem ein schonender Injektionsvorgang insbesondere zu Beginn der Injektion mit einfachen Mitteln erzielbar ist.

Dieses Problem wird erfindungsgemäß dadurch gelöst, dass das Dämpfungs-Gegenelement nachgiebig und insbesondere elastisch nachgiebig, und das Kopplungs- und Dämpfungselement im wesentlichen starr ausgebildet ist und zumindest teilweise mit dem Dämpfungs-Gegenelement in einem flexiblen Formeingriff steht und dieser ortsverändernd im Verlauf der Vorschubbewegung des Injizierkolbens bestehen bleibt und dabei zur Dämpfung eine Formänderungskraft wirksam wird.

Dadurch wird erreicht, dass vom Beginn des Injiziervorganges an auch unmittelbar nach der Entkopplung eine derartige Dämpfung einsetzt, dass ein zu schnelles Injizieren zu Beginn des Injiziervorganges vermieden wird. Dadurch ist es möglich, durch diesen schonenden Injiziervorgang Hämatome oder andere Gewebe- bzw. Gefäßirritationen zu vermeiden.

Dabei kann die Kopplungsstelle, wo der Widerstand zu überwinden ist, entweder vom Formeingriff selbst gebildet sein. Es kann aber auch vorgesehen sein, dass die Kopplungsstelle, wo der Widerstand zu überwinden ist, von einer Anlagestelle stirnseitig am Dämpfungs-Gegenelement, insbesondere einer Schulter desselben gebildet ist, wonach in Antriebsrichtung stromab dieser Kopplungsstelle der Formeingriff entsteht. In beiden Fällen bedingt der frühe Formeingriff einen zu heftigen anfänglichen Injiziervorgang.

Zweckmäßiger Weise kann stromab der Kopplungsstelle die Formänderungskraft im Verlaufe der Vorschubbewegung des Injizierkolbens zunächst zunehmen, um eine möglicherweise doch noch etwas zu geringe Anfangsdämpfung zu beeinflussen. Für die Zunahme der Formänderungskraft kann das Dämpfungs-Gegenelement zur Begrenzung der Formänderung im Anfangsbereich hinterstützt sein.

Es kann die Kopplungsstelle vom Formeingriff zusammen mit mindestens einer Schulter oder mindestens einem Wulst gebildet sein, die, bzw. der sich radial außerhalb des Dämpfungs-Gegenelements am Gehäuse oder an mindestens einer Mitnahmelasche befinden, die im Gehäuse gleitend bewegbar angeordnet sind, bzw. ist. Insbesondere kann die mindestens eine Mitnahmelasche Bestandteil einer Mitnahmehülse sein, die im Kopplungszustand vor dem Injizieren radial nach außen abgestützt ist und nachgiebig ist, und nach dem Entkoppeln radial nach außen ausweichbar gestaltet ist.

Zur Vergleichmäßigung des Injiziervorganges kann die Formänderungskraft im Verlaufe der Vorschubbewegung des Injizierkolbens nach der Zunahme wieder abnehmen, vorzugsweise kontinuierlich.

Für die Änderung der Formänderungskraft kann die Materialstärke des Dämpfungs-Gegenelements variieren.

Bevorzugt ist das Dämpfungs-Gegenelement eine Hülse oder ein Schlauch und das Kopplungs- und Dämpfungselement ein daran angepasstes Innenstück, das zugleich Abtriebselement ist oder mit dem Abtriebselement in Verbindung steht. Dieses Innenstück dringt mit dem gesamten Außenrand oder zumindest Teilen davon auf die Innenwand der Hülse und verformt diese zur Bildung des Formeingriffs, welcher bei der Bewegung des Innenstücks zusammen mit der Injizierkolbenbewegung aufrecht erhalten bleibt, wobei stetig eine Formänderung vorgenommen wird, so dass zur Dämpfung der Vorschubbewegung durch die Formänderung eine Gegenkraft erzeugt wird.

Insbesondere soll das Kopplungs- und Dämpfungselement zumindest teilweise scheibenförmig ausgebildet sein, deren Schmalseite quer zur Hülsenachse weist und insbesondere abgerundet ist, damit ein besseres Gleiten des Innenstücks an der Innenwand der Hülse gewährleistet ist.

Zur Beeinflussung der Flexibilität der Hülse kann diese nur am stromaufseitigen Endbereich am Gehäuse oder der Mitnahmehülse festgelegt sein, so dass eine Materialverdrängung sowohl in Axialrichtung als auch in radialer Richtung möglich ist. Dadurch ist es in größerem Umfang möglich, die Dämpfung für die Vorschubbewegung des Injizierkolbens unter Berücksichtigung der anderen Bewegungswiderstände und des Herausdrängens des Produktes aus der Injiziernadel einzustellen.

Für die Beeinflussung und insbesondere die Abnahme der Formänderungskraft wird die Wandstärke der Hülse in Antriebsrichtung abnehmend gestaltet.

Die Wandstärke der Hülse kann allerdings in Antriebsrichtung auch gleichbleibend sein. Dann jedoch soll eine radial außerhalb befindliche Gehäusewand konisch sich erweiternd derartig ausgebildet sein, dass in Antriebsrichtung ein zunehmender Abstand zwischen der Gehäusewand und der Hülse besteht, so dass aufgrund des Formeingriffes die Hülse radial sich zunehmend leichter verformen lässt, da die Hülse in radialer Richtung zunehmend ausweichen kann.

Die Wandstärke der Hülse kann dadurch abnehmen, dass die Innenwand und/oder die Außenwand der Hülse sich konisch erweiternd ausgebildet ist.

Vorzugsweise ist das Material des Dämpfungs-Gegenelements, insbesondere der Hülse, ein solches niedriger Shore Härte, insbesondere Gummi, Silikon, Thermoplaste wie TPU oder thermoplastischer Kautschuk.

Eine weitere parallele Lösung des Problems besteht darin, dass in Antriebsrichtung in einem kurzen Abstand stromab der Kopplungsstelle, wo der Widerstand zu überwinden ist, an einer Dämpfungskraft-Erhöhungsstelle eine erhöhte Dämpfungskraft bzw. über eine relativ kurze Wegstrecke eine zunehmende Dämpfungskraft der Injizierbewegung des Injizierkolbens vorhanden ist.

Auch dadurch wird erreicht, dass nach dem Überwinden des Widerstandes und somit nach dem Entkoppeln der anfängliche Injiziervorgang infolge einer erhöhten Dämpfung reduziert ist und somit der Injiziervorgang schonender erfolgt.

Bei dieser Lösungsvariante ist vorzugsweise das Kopplungs-und Dämpfungselement ein nachgiebiges und vorzugsweise elastisch nachgiebiges Element, und das Dämpfungs-Gegenelement ein starres Element, an dem entlang das nachgiebige Element gleitet. Dabei ist die Gleitfläche stromab der Kupplungsstelle zunächst ansteigend ausgebildet.

Um im weiteren Verlauf der Vorschubbewegung des Injizierkolbens den Injektionsvorgang zu Vergleichmäßigen, nimmt die Dämpfungskraft von der Dämpfungskraft-Erhöhungsstelle im Verlaufe der Injizierkolbenbewegung ab, wozu die Gleitfläche nach der Erhöhung abfallend ausgebildet ist.

### Figurenbeschreibung

Ausführungsbeispiele der Erfindung sind rein schematisch in den beigefügten Zeichnungen dargestellt. Die Zeichnungen zeigen:
Fig. 1 eine perspektivische Darstellung eines Autoinjektionsgerätes als sogenannter Injektionspen;
Fig. 2 eine Längsschnittdarstellung durch das Autoinjektionsgerät gemäß Fig. 1;

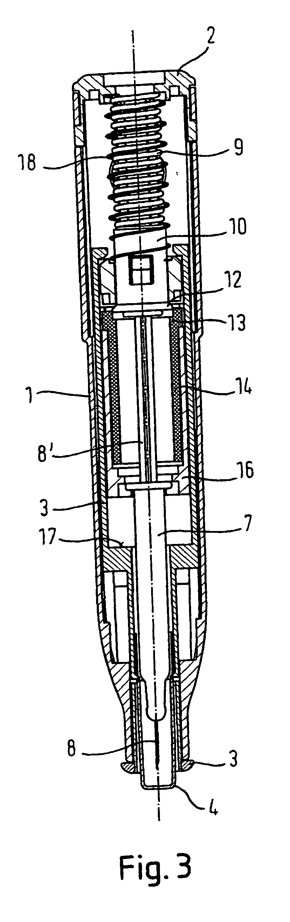

Fig. 3 eine Längsschnittdarstellung ähnlich der der Fig. 2, jedoch entsprechend einem senkrecht dazu durchgeführten Längsschnitt;
Fig. 4 eine Schnittansicht nur eines Teils des Autoinjektionsgerätes;
Fig. 5 eine Seitenansicht eines Autoinjektionsgerätes einer anderen Ausführungsform;
Fig. 6a eine Schnittansicht entlang der Linie B-B in Fig. 5;
Fig. 6b eine Teilansicht entsprechend dem Kreis C in Fig. 6a.

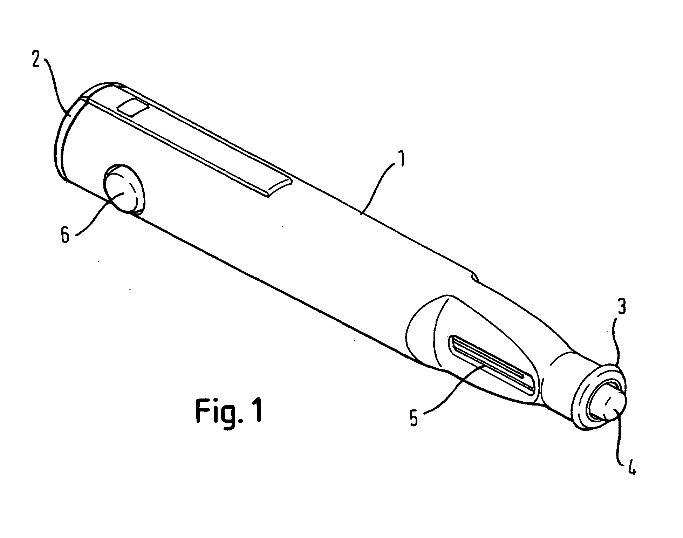

Hinsichtlich des äußeren Erscheinungsbildes ist das erfindungsgemäße Injektionsgerät der Fig. 1 in perspektivischer Ansicht entnehmbar. Dieses Autoinjektionsgerät weist ein Gehäuse 1 mit einem Deckel 2 auf, der am rückwärtigen Ende des Gehäuses aufgesetzt ist. Am vorderen Ende des Gehäuses 1 befindet sich eine bewegliche Auslösehülse 3 und eine Schutzkappe 4 zum Schutz der Injektionsnadel die hier nicht sichtbar ist. Des weiteren ist im vorderen Endbereich ein Sichtfenster 5 dargestellt. Im rückwärtigen Endbereich des in Fig. 1 dargestellten Autoinjektionsgerätes befindet sich ein Entriegelungsdruckknopf 6.

Der Auslösemechanismus als solcher ist nicht Gegenstand der vorliegenden Erfindung, da der Auslösemechanismus ähnlich gestaltet ist wie in der WO 2004/098687 A1 der Anmelderin, weswegen dieser Mechanismus nur am Rande beschrieben wird. Im Übrigen wird auf diese Veröffentlichung Bezug genommen.

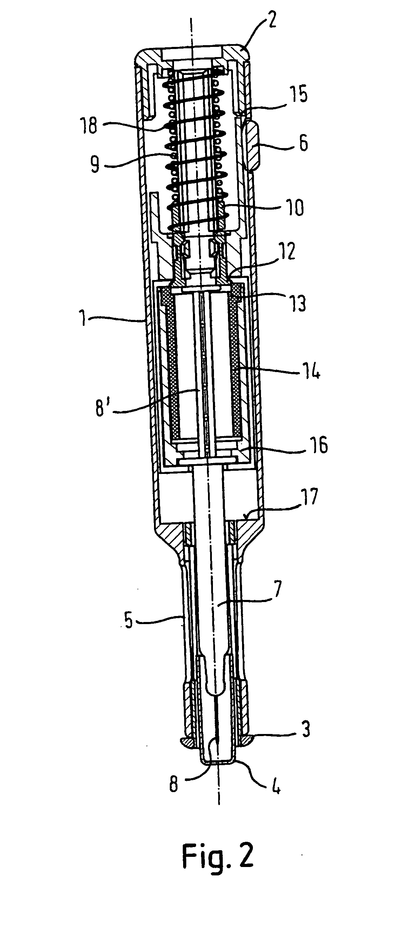

Innerhalb des Gehäuses (Fig. 2) befindet sich ein Behälter 7, der üblicherweise ein Glasbehälter ist, in dem sich das zu injizierende Produkt befindet. Im vorderen Ende des Behälters 7 ist eine Injektionsnadel 8 angebracht, die von der Schutzkappe 4 schützend abgedeckt ist, wobei im Ausgangszustand des Autoinjektionsgerätes die Injektionsnadel sich vollständig in der Auslösehülse 3 befindet. Innerhalb des Behälters 7 befindet sich ein hier nicht dargestellter Injizierkolben, mit dem beim Injizieren das Produkt aus dem Behälter 7 durch die Injiziernadel 8 in den Körper eines Patienten eingespritzt wird. Dieser Kolben sitzt an einer Kolbenstange 8'.

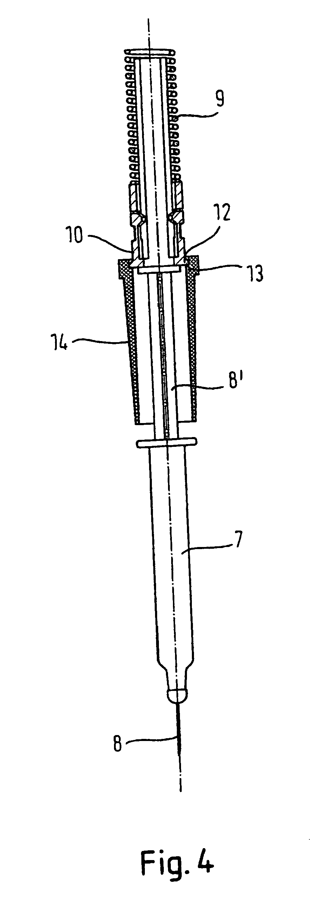

Am Deckel 2 stützt sich als Antriebselement eine Druckfeder 9 ab, die mit dem anderen Ende auf ein Abtriebselement einwirkt. Dieses Abtriebselement ist zugleich ein Innenstück 10. Das Innenstück 10 weist am stromabliegenden Ende eine scheibenförmige Ausbildung 12 aufweist. Der Rand dieser scheibenförmigen Ausbildung 12 liegt wiederum an einer Schulter 13 einer Dämpfungshülse bzw. eines Dämpfungsschlauchs (Schlauch 14) an, wie dies insbesondere aus Fig. 4 ersichtlich ist.

Das Innenstück 10 besteht aus einem starren Material und der Schlauch 14 aus einem Material niedriger Shore-Härte, insbesondere Gummi, Silikon, Thermoplaste wie TPU oder thermoplastischer Kautschuk.

Das Autoinjektionsgerät der so beschriebenen Art funktioniert wie folgt, wobei wie gesagt der automatische Auslösemechanismus im Hinblick auf die WO 2004/098687 A1 nur am Rande erläutert wird:

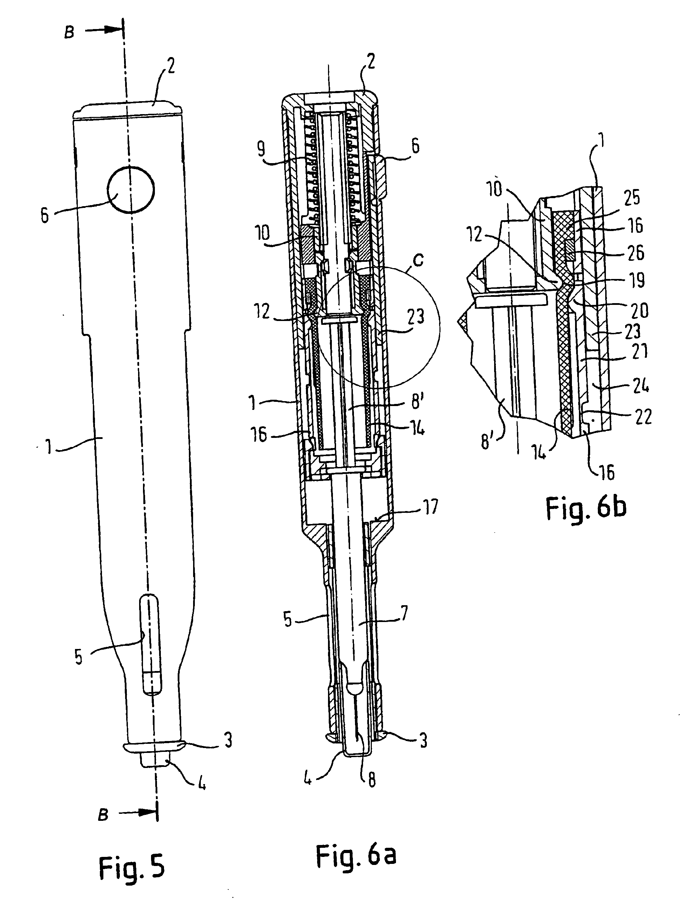

Zunächst einmal wird die Auslösehülse 3 aus dem Gehäuse in Axialrichtung heraus gezogen, wodurch Mitnehmer die Schutzkappe 4 mitnehmen und abwerfen. Dies bewerkstelligt ein Freigeben der Injektionsnadel 8 und ein Scharfmachen des Autoinjektionsgerätes, wobei die Auslösehülse 3 die Spitze der Injektionsnadel 8 überragt. Wenn nun ein Injiziervorgang eingeleitet werden soll, so drückt der Patient den Entriegelunsdruckknopf 6 ein, wodurch eine Verriegelung 15 (Fig. 2) gelöst wird. Während der Druckknopf 6 eingedrückt gehalten wird, wird das äußere Ende der Auslösehülse 3 gegen die Haut des Patienten gedrückt, wodurch die Antriebskraft der Feder 9 ausgelöst wird. Die Feder wirkt nun über das Abtriebselement sowie das Innenstück 10, die scheibenförmige Ausbildung 12, die Schulter 13 und eine Schiebehülse 16 auf den Behälter 7, so dass ein Einstechen der Injiziernadel 8 in die Haut des Patienten erfolgt, bis ein Anschlagen an einem Anschlag 17 erfolgt. Durch dieses Anschlagen überwindet die scheibenförmige Ausbildung 12 des Innenstücks 10 die Schulter 13 des Schlauches 14, der am oberen Ende der Schiebehülse 16 befestigt ist. Nach Überwinden der Schulter 13 als Kopplungselement zwischen Innenstück 10 und Schlauch 14 dringt die scheibenförmige Ausbildung 12 mit ihrem Rand verformend in das Schlauchmaterial ein und bildet einen Formeingriff 19, wie er beispielsweise auch aus Fig. 6b erkennbar ist. Durch diesen Formeingriff 19 erfolgt eine Dämpfung der Injizierbewegung des nicht dargestellten Injizierkolbens derart, beispielsweise durch Hinterstützen des Schlauches zu Beginn der Bewegung nach der Entkopplung, so dass der anfängliche Injiziervorgang so sanft erfolgt, dass keine Hämatome entstehen können. Während der Vorschubbewegung des Injizierkolbens bleibt dieser Formeingriff erhalten, verändert allerdings seine Ortslage mit zunehmender Injizierkolbenbewegung, da der Schlauch 14 radial nach außen und bei einseitiger Befestigung auch in Axialrichtung sich verformen kann. Diese Verformungskraft bewirkt die Dämpfung der Injizierbewegung und wenn beispielsweise in Vorschubrichtung des Injizierkolbens die Wandstärke des Schlauches abnimmt, stellt sich eine Kompensation der abnehmenden Federkraft der Feder 9 ein, so dass mit einer gesteuerten Dämpfung ein kontinuierliches, gleichbleibendes Injizieren erfolgen kann.

Es ist auch möglich, die Wandstärke des Schlauches gleichbleibend zu gestalten, aber die Verschiebehülse 16 in Vorschubrichtung des Injizierkolbens konisch sich erweiternd zu gestalten, so dass der Schlauch mit zunehmender Vorschubbewegung stärker radial ausweichen kann.

Wenn der Anfangsbereich des Schlauches am stromaufseitigen Ende der Verschiebehülse 16 radial hinterstützt ist, so kann man auf besonders einfache und wirkungsvolle Weise die Anfangsdämpfung nach der Entkopplung derart beeinflussen, dass wirkungsvoll ein zu starkes Injizieren zu Beginn des Injiziervorganges vermieden werden kann.

Eine schwächere Feder 18, die die Antriebsfeder 9 umgibt, hat den Zweck, nach dem Injiziervorgang die Auslösehülse 3 wieder auszufahren, so dass die Injiziernadel zur Verhinderung von Verletzungen wieder innerhalb der Auslösehülse 3 verschwindet.

Die in Fig. 5 bis 6 dargestellte Ausführungsform unterscheidet sich wesentlich von der erstbeschriebenen Ausführungsform durch Einzelmerkmale, die nachfolgend erläutert werden. Im Übrigen werden wegen Übereinstimmungen Beschreibungswiederholungen vermieden und auf die erste Ausführungsform Bezug genommen.

Die zu überwindende Kopplung ist bei dieser Ausführungsform durch den Formeingriff 19 zwischen der scheibenförmigen Ausbildung 12 und dem Schlauch 14 gebildet, wie dies in Fig. 6b aber auch in Fig. 6a dargestellt ist. In der Ausgangslage besteht bereits dieser Formgriff als Kopplungszustand, unterstützt durch mindestens einen Wulst 20, der an einer Lasche 21 ausgebildet ist. Diese Lasche 21 sitzt in einer Ausnehmung der Verschiebehülse 16 und ist um eine Schwachstelle 22 als Gelenk an der Verschiebehülse 16 befestigt, im Übrigen aber frei beweglich. Diese Lasche 21 stützt sich in der Ausgangslage entweder direkt am Gehäuse 1 ab, bevorzugt aber an einem hülsenförmigen Teil 23, das bevorzugt am Deckel 2 befestigt ist, so dass zwischen der Lasche 21 und der Innenwand des Gehäuses 1 ein Freiraum 24 verbleibt, der folgende Bewandtnis hat.

Durch die Kopplung am Wulst 20 verschieben sich die Teile 10, 12, 14, 16 zusammen mit dem Behälter 7 und der Injiziernadel 8 in Injizierrichtung zum Einstechen der Injiziernadel 8. Die Lasche 21 wird über den Wulst 20 und den Formeingriff 19 an das hülsenförmige Teil 3 gedrückt, so dass eine gewisse Dämpfung beim Einstechen erfolgt. Am Ende des Injiziervorganges und vorzugsweise noch vor dem Anschlagen an dem Anschlag 17 gelangt das freie Ende der Lasche 21 in den Freiraum 24, so dass der Wulst 20 radial nach außen ausweicht und dadurch die Entkopplung erfolgen kann, mit der der Injiziervorgang eingeleitet wird auf die bereits zuvor beschriebene Weise. Dadurch kann auch verhindert werden, dass beim Auftreffen auf einen harten Gegenstand, beispielsweise Knochenmasse, innerhalb der Haut des Patienten bereits der Injiziervorgang ausgelöst wird.

Damit der Schlauch 14 nicht innerhalb der Verschiebehülse 16 durchrutschen kann, stützt sich dieser mit einem stromauf liegenden Bund 25 an einem Ring 26 ab und somit an der Verschiebehülse 16 ab. Diese Verschiebehülse kann sich beim Injizieren nicht mehr bewegen.

Anstelle von vorzugsweise zwei Laschen 21 mit jeweils einem Wulst 20 kann auch ein Ringwulst vorgesehen sein, allerdings dann mit einer Schlitzung.

## Patentansprüche

1. Autoinjektionsgerät, insbesondere zum Injizieren von Produkten in den Körper eines Lebewesens, mit
- einem Gehäuse (1),
- einem darin befindlichen, das zu injizierende Produkt enthaltenden Behälter (7), der mit einer Injiziernadel (8) in Verbindung steht und einen Injizierkolben enthält,
- eine Antriebseinrichtung mit wenigstens einem Antriebselement (9) und wenigstens einem Abtriebselement, wobei das Antriebselement (9) auf das Abtriebselement und das Abtriebselement auf den Injizierkolben und/oder den Behälter (7) mit der daran befindlichen Injiziernadel (8) einwirkt,
- einer Dämpfungseinrichtung zur Dämpfung der Einstechbewegung und/oder der Injizierbewegung des Injizierkolbens,
- wobei das Abtriebselement bis zum Erreichen einer vorderen Position des Behälters (7) vom Injizierkolben vorschubmäßig entkoppelt und über ein Kopplungs- und Dämpfungselement (10) in Antriebsrichtung mittelbar oder unmittelbar mit dem Behälter (7) gekoppelt ist, indem das Kopplungs-und Dämpfungselement (10) zur Entkopplung an einer Kopplungsstelle (13) einen Widerstand überwinden muss, und bei Erreichen der vorderen Position mit dem Injizierkolben vorschubmäßig gekoppelt wird,
- und wobei das Kopplungs- und Dämpfungselement (10) zur Dämpfung mit einem Dämpfungs-Gegenelement (14) zusammen wirkt, wobei das Kopplungs- und Dämpfungselement (10) im Wesentlichen starr ausgebildet ist
**dadurch gekennzeichnet, dass**
das Dämpfungs-Gegenelement (14) nachgiebig und insbesondere elastisch nachgiebig ausgebildet ist und das Kopplungs- und Dämpfungselement (10) zumindest teilweise mit dem Dämpfungs-Gegenelement (14) in einem flexiblen Formeingriff (19) steht und dieser ortsverändernd im Verlaufe der Vorschubbewegung des Injizierkolbens bestehen bleibt und dabei zur Dämpfung eine Formänderungskraft wirksam wird.

2. Autoinjektionsgerät nach Anspruch 1, **dadurch gekennzeichnet, dass** die Kopplungsstelle, wo der Widerstand zu überwinden ist, vom Formeingriff (19) gebildet ist.

3. Autoinjektionsgerät nach Anspruch 1, **dadurch gekennzeichnet, dass** die Kopplungsstelle, wo der Widerstand zu überwinden ist, von einer Anlagestelle (13) stirnseitig am Dämpfungs-Gegenelement (14), insbesondere einer Schulter desselben gebildet ist, wonach in Antriebsrichtung stromab dieser Kopplungsstelle der Formeingriff (19) entsteht.

4. Autoinjektionsgerät nach Anspruch 2, **dadurch gekennzeichnet, dass** die Kopplungsstelle vom Formeingriff (19) zusammen mit mindestens einer Schulter oder mindestens einem Wulst (20) gebildet ist, die, bzw. der sich radial außerhalb des Dämpfungs-Gegenelementes (14) am Gehäuse (1) oder an mindestens einer Mitnahmelasche (21) befindet, die im Gehäuse (1) bewegbar angeordnet ist.

5. Autoinjektionsgerät nach Anspruch 4, **dadurch gekennzeichnet, dass** die mindestens eine Mitnahmelasche (21) im Kopplungszustand vor dem Injizieren radial nach außen abgestützt und unnachgiebig ist und nach dem Entkoppeln radial nach außen ausweichbar gestaltet ist.

6. Autoinjektionsgerät nach Anspruch 3, **dadurch gekennzeichnet, dass** stromab der Kopplungsstelle die Formänderungskraft im Verlaufe der Vorschubbewegung des Injizierkolbens zunächst zunimmt.

7. Autoinjektionsgerät nach Anspruch 6, **dadurch gekennzeichnet, dass** für die Zunahme der Formänderungskraft das Dämpfungs-Gegenelement (14) zur Begrenzung der Formänderung hinterstützt ist.

8. Autoinjektionsgerät nach einem der Ansprüche 1 bis 7, **dadurch gekennzeichnet, dass** die Formänderungskraft im Verlauf der Vorschubbewegung des Injizierkolbens nach der Zunahme wieder abnimmt.

9. Autoinjektionsgerät nach einem der Ansprüche 3 bis 8, **dadurch gekennzeichnet, dass** zur Änderung der Formänderungskraft die Materialstärke des Dämpfungs-Gegenelementes (14) variiert.

10. Autoinjektionsgerät nach einem der Ansprüche 1 bis 7, **dadurch gekennzeichnet, dass** das Dämpfungs-Gegenelement eine Hülse (14) oder ein Schlauch (14) und das Kopplungs- und Dämpfungselement ein an die Hülse oder den Schlauch angepasstes Innenstück (10) ist, welches zugleich Abtriebselement ist oder mit dem Abtriebselement in Verbindung steht.

11. Autoinjektionsgerät nach Anspruch 10, **dadurch gekennzeichnet, dass** das Kopplungs- und Dämpfungselement (10) zumindest teilweise scheibenförmig (12) ausgebildet ist, deren Schmalseite quer zur Hülsenachse weist und insbesondere abgerundet ist.

12. Autoinjektionsgerät nach Anspruch 5, 8 und 9, **dadurch gekennzeichnet, dass** die Hülse (14) oder der Schlauch (14) nur am stromaufseitigen Endbereich am Gehäuse (1) oder einer Mitnehmerhülse (16) festgelegt ist.

13. Autoinjektionsgerät nach einem der Ansprüche 1 bis 12, **dadurch gekennzeichnet, dass** die Wandstärke der Hülse (14) in Antriebsrichtung abnimmt.

14. Autoinjektionsgerät nach einem der Ansprüche 8 bis 12, **dadurch gekennzeichnet, dass** die Wandstärke der Hülse (14) in Antriebsrichtung gleichbleibend ist und diesbezüglich eine radial außerhalb befindliche Gehäusewand konisch sich erweiternd derart ausgebildet ist, dass in Antriebsrichtung ein zunehmender Abstand zwischen Gehäusewand und Hülse besteht.

15. Autoinjektionsgerät nach Anspruch 13, **dadurch gekennzeichnet, dass** die Wandstärke der Hülse (14) **dadurch** abnimmt, dass die Innenwand und/oder die Außenwand der Hülse sich konisch erweiternd ausgebildet ist.

16. Autoinjektionsgerät nach einem der Ansprüche 1 bis 15, **dadurch gekennzeichnet, dass** das Material des Dämpfungs-Gegenelementes (14), insbesondere der Hülse, ein solches niedriger Shore Härte ist, insbesondere Gummi, Silikon, Thermoplaste wie TPU oder thermoplastischer Kautschuk.

## Claims

1. Automatic injection device, in particular for injection of products into the body of a living thing, having
- a housing (1),
- a container (7) located therein containing the product to be injected and which is connected to an injection needle (8) and contains a injection plunger,
- a drive device having at least one drive element (9) and at least one driven element, wherein the drive element (9) acts on the driven element and the driven element acts on the injection plunger and/or the container (7) with the injection needle (8) located therein,
- a damping device for damping the puncturing movement and/or the injection movement of the injection plunger,
- wherein the driven element is uncoupled from the injection plunger according to advance by the time it reaches front position of the container (7) and is coupled indirectly or directly to the container (7) via a coupling and damping element (10) in drive direction, in that the coupling and damping element (10) has to overcome a resistance for uncoupling at a coupling point (13), and on reaching the front position, is coupled to the injection plunger according to advance,
- and wherein the coupling and damping element (10) cooperates with a damping counter-element (14) for damping, wherein the coupling and damping element (10) is designed to be essentially rigid,
**characterised in that** the damping counter-element (14) is designed to be flexible and in particular resiliently flexible, and the coupling and damping element (10) is in flexible positive engagement at least partly with the damping counter-element (14) and the latter remains movable in the course of the advance movement of the injection plunger and thus a deformation force becomes active for damping.

2. Automatic injection device according to claim 1, **characterised in that** the coupling point where the resistance is to be overcome, is formed by the positive engagement (19).

3. Automatic injection device according to claim 1, **characterised in that** the coupling point where the resistance is to be overcome, is formed by a support point (13) on the end-face side of the damping counter-element (14), in particular a shoulder of the same, after which in drive direction downstream of this coupling point, the positive engagement (19) is produced.

4. Automatic injection device according to claim 2, **characterised in that** the coupling point is formed by the positive engagement (19) together with at least one shoulder or at least one bead (20), which is located radially outside of the damping counter-element (14) on the housing (1) or on at least one drive plate (21), which is arranged to be movable in the housing (1).

5. Automatic injection device according to claim 4, **characterised in that** the at least one drive plate (21) in the coupling state before injection, is supported radially outwards and is not flexible and after uncoupling, is designed to be yieldable radially outwards.

6. Automatic injection device according to claim 3, **characterised in that** downstream of the coupling point, the deformation force in the course of the advance movement of the injection plunger first of all increases.

7. Automatic injection device according to claim 6, **characterised in that** for the increase of the deformation force, the damping counter-element (14) is supported from the rear to limit the deformation.

8. Automatic injection device according to one of claims 1 to 7, **characterised in that** the deformation force in the course of the advance movement of the injection plunger decreases again after the increase.

9. Automatic injection device according to one of claims 3 to 8, **characterised in that** to change the deformation force, the material thickness of the damping counter-element (14) varies.

10. Automatic injection device according to one of claims 1 to 7, **characterised in that** the damping counter-element is a sleeve (14) or a tube (14) and the coupling and damping element is an inner piece (10) adapted to the sleeve or the tube and which at the same time is driven element or is connected to the driven element.

11. Automatic injection device according to claim 10, **characterised in that** the coupling and damping element (10) is designed to be at least partly disc-like (12), the narrow side of which points transversely to the sleeve axis and in particular is rounded.

12. Automatic injection device according to claim 5, 8 and 9, **characterised in that** the sleeve (14) or the tube (14) is fixed only at the upstream-side end region to the housing (1) or a driver sleeve (16).

13. Automatic injection device according to one of claims 1 to 12, **characterised in that** the wall thickness of the sleeve (14) decreases in drive direction.

14. Automatic injection device according to one of claims 8 to 12, **characterised in that** the wall thickness of the sleeve (14) is constant in drive direction and with regard to this, a housing wall located radially outside is designed to be conically expanding such that in drive direction, an increasing distance exists between housing wall and sleeve.

15. Automatic injection device according to claim 13**, characterised in that** the wall thickness of the sleeve (14) decreases **in that** the inner wall and/or the outer wall of the sleeve is designed to be conically expanding.

16. Automatic injection device according to one of claims 1 to 15, **characterised in that** the material of the damping counter-element (14), in particular the sleeve, is one of low Shore hardness, in particular rubber, silicone, thermoplastics, such as TPU or thermoplastic rubber.

## Revendications

1. Dispositif d'auto-injection, en pariculier pour injecter des produits dans le corps d'un être vivant, avec :
- un boîtier (1),
- un récipient (7), situé à l'intérieur de celui-ci, contenant le produit à injecter, le récipient étant relié à une aiguille d'injection (8) et contenant un piston d'injection,
- un dispositif d'entraînement, comprenant au moins un élément menant (9) et au moins un élément mené, l'élément menant (9) agissant sur l'élément mené et l'élément mené agissant sur le piston d'injection et/ou le récipient (7) avec l'aiguille d'injection (8) s'y trouvant,
- un dispositif d'amortissement, pour amortir le déplacement de piquage et/ou le déplacement d'injection du piston d'injection,
- l'élément mené étant désaccouplé du piston d'injection, en terme d'avancement, jusqu'à l'atteinte d'une position avant du récipient (7), et étant couplé, indirectement ou directement, au récipient (7), dans le sens de l'entraînement, par l'intermédiaire d'un élément de couplage et d'amortissement (10), par le fait que l'élément de couplage et d'amortissement (10) doit surmonter une résistance en un point de couplage (13) pour obtenir le désaccouplement, et est couplé au piston d'injection, en terme d'avancement, à l'atteinte de la position avant, et l'élément de couplage et d'amortissement (10) coopérant, pour l'amortissement, avec un contre-élément d'amortissement (14), l'élément de couplage et d'amortissement (10) étant réalisé de façon sensiblement rigide,
**caractérisé en ce que**
le contre-élément d'amortissement (14) est déformable et, en particulier, est déformable élastiquement, et l'élément de couplage et d'amortissement (10) étant placé dans une mise en prise flexible au moins partielle, de par la forme, avec le contre-élément d'amortissement (14), et cette mise en prise persistant, avec un changement d'emplacement, au cours du déplacement d'avancement du piston d'injection et une force de modification de forme agissant alors pour assurer l'amortissement.

2. Dispositif d'auto-injection selon la revendication 1, **caractérisé en ce que** le point de couplage, où la résistance doit être surmontée, est formé par la mise en prise par la forme (19).

3. Dispositif d'auto-injection selon la revendication 1, **caractérisé en ce que** le point de couplage, où la résistance doit être surmontée, est formé par un point d'appui (13) situé frontalement sur le contre-élément d'amortissement (14), en particulier un épaulement de celui-ci, suite à quoi la mise en prise par la forme (19) est créée en aval de ce point de couplage, en observant dans la direction d'entraînement.

4. Dispositif d'auto-injection selon la revendication 2, **caractérisé en ce que** point de couplage est formé par la mise en prise par la forme (19), conjointement avec au moins un épaulement ou au moins un bourrelet (20), épaulement ou bourrelet se trouvant radialement à l'extérieur du contre-élément d'amortissement (14) sur le boîtier (1), ou en au moins une patte d'entraînement (21) disposée de façon déplaçable dans le boîtier (1).

5. Dispositif d'auto-injection selon la revendication 4, **caractérisé en ce que,** à l'état de couplage avant l'injection, la au moins une patte d'entraînement (21) est soutenue radialement vers l'extérieur et n'est pas déformable et, après le désaccouplement, est conformée de manière à pouvoir s'échapper radialement vers l'extérieur.

6. Dispositif d'auto-injection selon la revendication 3, **caractérisé en ce que**, en aval du point de couplage, la force de modification de forme commence par augmenter au cours du déplacement d'avancement du piston d'injection.

7. Dispositif d'auto-injection selon la revendication 6, **caractérisé en ce que** l'augmentation de la force de modification de forme soutient par l'arrière le contre-élément d'amortissement (14), afin de limiter la modification de forme.

8. Dispositif d'auto-injection selon l'une quelconque des revendications 1 à 7, **caractérisé en ce que**, après l'augmentation, la force de modification de forme diminue de nouveau au cours du déplacement d'avancement du piston d'injection.

9. Dispositif d'auto-injection selon l'une quelconque des revendications 3 à 8, **caractérisé en ce que** l'épaisseur de matériau du contre-élément d'amortissement (14) varie, afin de modifier la force de modification de forme.

10. Dispositif d'auto-injection selon l'une quelconque des revendications 1 à 7, **caractérisé en ce que** le contre-élément d'amortissement est une douille (14) ou un tuyau (14), et l'élément de couplage et d'amortissement est une pièce intérieure, adaptée à la douille ou au tuyau(10), pièce intérieure qui est en même temps l'élément mené ou est reliée à l'élément mené.

11. Dispositif d'auto-injection selon la revendication 10, **caractérisé en ce que** l'élément de couplage et d'amortissement (10) est réalisé, au moins partiellement, en forme de disque (12), dont le côté étroit est tourné transversalement à l'axe de douille et en particulier est arrondi.

12. Dispositif d'auto-injection selon les revendications 5, 8 et 9, **caractérisé en ce que** la douille (14) ou le tuyau (14) est fixé(e), uniquement sur la zone d'extrémité située côté amont, sur le boîtier (1) ou une douille d'entraînement 16).

13. Dispositif d'auto-injection selon l'une quelconque des revendications 1 à 12, **caractérisé en ce que** l'épaisseur de paroi de la douille (14) diminue en évoluant dans le sens d'entraînement.

14. Dispositif d'auto-injection selon l'une quelconque des revendications 8 à 12, **caractérisé en ce que** l'épaisseur de paroi de la douille (14) est constante en évoluant dans le sens d'entraînement et, à ce sujet, une paroi de boîtier, se trouvant radialement à l'extérieur, est conformée de manière conique, en s'élargissant de manière qu'il y ait, entre paroi de boîtier et douille, un espacement qui aille en augmentant en évoluant dans le sens d'entraînement.

15. Dispositif d'auto-injection selon la revendication 13, **caractérisé en ce que** l'épaisseur de paroi de la douille (14) diminue par le fait que la paroi intérieure et/ou la paroi extérieure de la douille est conformée en s'élargissant de manière conique.

16. Dispositif d'auto-injection selon l'une quelconque des revendications 1 à 15, **caractérisé en ce que** le matériau du contre-élément d'amortissement (14), en particulier de la douille, est un matériau de faible dureté Shore, en particulier du caoutchouc, du silicone, un thermoplastique tel que du TPU ou un caoutchouc thermoplastique.
